# EUROPÄISCHE PATENTSCHRIFT

(11) **EP 3 344 333 B1**
(45) Veröffentlichungstag und Bekanntmachung des Hinweises auf die Patenterteilung: **09.12.2020**
(21) Anmeldenummer: 16758185.9
(22) Anmeldetag: 31.08.2016
(51) Int. Cl.: A61N 2/00, A61N 2/02

(54) **SPULENANORDNUNG UND SYSTEM ZUR TRANSKRANIELLEN MAGNETSTIMULATION**
COIL DEVICE AND SYSTEM FOR TRANSCRANIAL MAGNETIC STIMULATION
BOBINE ET SYSTÈME POUR STIMULATION MAGNÉTIQUE TRANSCRANIENNE

(30) Priorität: 31.08.2015 DE 102015114483
(43) Veröffentlichungstag der Anmeldung: 11.07.2018
(73) Patentinhaber: Eberhard Karls Universität Tübingen Medizinische Fakultät, 72074 Tübingen (DE)
(72) Erfinder: ZRENNER, Christoph, 72076 Tübingen (DE); ZIEMANN, Ulf, 72074 Tübingen (DE)
(74) Vertreter: Witte, Weller & Partner Patentanwälte mbB
(86) Internationale Anmeldenummer: PCT/EP2016/070499
(87) Internationale Veröffentlichungsnummer: WO 2017/037107

(56) Entgegenhaltungen:
- WO-A2-2008/070001
- US-A1- 2002 007 128
- US-B1- 9 095 266

## Beschreibung

Die vorliegende Erfindung betrifft das Gebiet der Medizintechnik und insbesondere das Gebiet der transkraniellen Magnetstimulation (TMS). Die vorliegende Erfindung bezieht sich auf eine Spulenanordnung mit einem Gehäuse und einer darin angeordneten Magnetspule zum Erzeugen eines Magnetfeldes zur transkraniellen Magnetstimulation, eine Elektrodenanordnung sowie ein System zur transkraniellen Magnetstimulation.

Transkranielle Magnetstimulation (TMS) ist eine Technik, um das menschliche Gehirn nichtinvasiv zu stimulieren. Insbesondere kann TMS eine Depolarisierung oder Hyperpolarisierung der Neuronen des Gehirns bewirken. TMS nutzt hierbei elektromagnetische Induktion, um durch ein sich zeitlich schnell veränderndes Magnetfeld schwache elektrische Ströme im Gehirn zu induzieren. Dies ermöglicht beispielsweise eine Aktivierung von spezifischen oder allgemeinen Hirnarealen mit minimaler Beeinträchtigung des Patienten bzw. Probanden. TMS nutzt elektromagnetische Induktion, um elektrische Energie durch die Kopfhaut und die Schädeldecke zu übertragen ohne dabei Schmerzen zu verursachen wie bei einer direkten perkutanen elektrischen Stimulation durch die Haut hindurch.

Zur TMS wird eine leitende Spule bzw. Magnetspule auf dem Kopf oder in unmittelbarer Nähe des Kopfes platziert und ein starker sich zeitlich schnell verändernder Strom durch die Spule geleitet. Dies bewirkt ein magnetisches Feld, welches im Wesentlichen ungedämpft und schmerzfrei durch die Schädeldecke und das Gewebe dringt. Um einen hinreichend starken Stromfluss zu induzieren, um Neuronen im Gehirn zu depolarisieren, startet und stoppt der Strom in der Spule üblicherweise innerhalb weniger hundert Mikrosekunden bzw. kehrt der Strom in der Spule seine Flussrichtung innerhalb weniger hundert Mikrosekunden um.

TMS wird derzeit in verschiedenen Formen angewandt. Bei der sogenannten Einzel-Puls TMS wird ein einzelner Puls von der Spule bereitgestellt. Bei der sogenannten repetitiven TMS (rTMS) wird ein Pulszug mit mehreren magnetischen Pulsen über eine definierte Zeitperiode bereitgestellt. Aktuell sind zahlreiche Stimulationsprotokolle mit verschiedenen Stimulationsmustern im Einsatz, beispielsweise werden bei intermittent Theta Burst Stimulation (iTBS) jeweils 3 Pulse im 10 Millisekunden Abstand ausgelöst, und diese Dreifachpulse 5 Mal pro Sekunde wiederholt, wobei nach mehreren Minuten Stimulation jeweils kurz pausiert wird. Die sich in der Anwendung befindenden Protokolle haben eine definierte Stimulationsabfolge, die vorprogrammiert/vordefiniert ist.

TMS kann beispielsweise in der medizinischen Forschung und zur therapeutischen Anwendung bei der Rehabilitation nach Schlaganfall, neuropathischen Schmerzen, Tinnitus, Depression oder Schizophrenie Anwendung finden.

Ein herkömmliches System zur transkraniellen Magnetstimulation besteht aus einer Spulenanordnung sowie einem Stimulusgenerator, welcher die Spulenanordnung speist. Die Spulenanordnung wird mitunter auch als "coil" bezeichnet, der Stimulusgenerator als "magnetische Stimulationseinheit", "Stimulator" oder "magnetic stimulator unit".

Derartige TMS Systeme sind bereits kommerziell verfügbar. Als Spulenanordnungen werden beispielsweise ringförmige Spulen oder achterförmige Spulen von der Firma The Magstim Company Ltd, UK (z.B. "Double 70mm Remote Control Coil") oder von der Firma MagVenture, USA ("MagPro" Spulen) angeboten. Als Stimulusgeneratoren sind beispielsweise das Modell Magstim Rapid von The Magstim Company Ltd, UK bzw. die MagPro Stimulatoren von MagVenture, USA zu nennen. Die Spulenanordnungen können über einen Schalter zum manuellen Triggern eines magnetischen Pulses durch den Stimulusgenerator verfügen. Ferner kann ein Stimulusgenerator einen Triggereingang zum externen Auslösen eines TMS-Pulses aufweisen.

US 2002/0007128 A1 lehrt, dass es aus Gründen der Patientensicherheit und zur Überwachung der Wirksamkeit der transkraniellen Magnetstimulation wünschenswert sein kann, während einer TMS Sitzung auch ein Elektroenzephalogramm (EEG) des Patienten zu überwachen. Für die EEG Messung wird neben dem TMS-System ein EEG-System bereitgestellt. EEG-Elektroden werden auf herkömmliche Art und Weise an der Kopfhaut des Patienten angebracht und mit einer EEG-Steuereinheit verbunden. Für die Positionierung der Elektroden auf der Kopfhaut nennt die US 2002/0007128 A1 die Anordnung gemäß dem internationalen 10-20 System.

WO 2014/139029 A1 schlägt für die Positionierung einer TMS-Spulenanordnung eine Positionierhilfe in Form einer Positionierhaube mit Führungselementen zur Ausrichtung vor, welche mit entsprechenden Ausrichtaufnahmeelementen einer TMS-Spulenanordnung zusammenwirken, um die Spulenanordnung in Bezug zur Positionierhilfe in einer gewünschten Position auf dem Kopf des Probanden zu fixieren. Die Positionierhilfe kann EEG-Elektroden aufweisen. In einem ersten Schritt wird die Positionierhilfe (ohne TMS-Spulenanordnung) am Schädel des Probanden ausgerichtet und ortsfest befestigt. In einem zweiten Schritt wird die separate TMS-Spulenanordnung an der Positionierhilfe ausgerichtet und befestigt. Eine Verschiebung der TMS-Spulenanordnung bezüglich des Schädels wird somit verhindert.

Die US 2002/0007128 A1 offenbart ferner, dass zwischen dem EEG-System und dem TMS-System ein Steuersystem vorgesehen ist, welches das TMS-System bei Bedarf abschalten kann. Ein Problem von TMS besteht darin, dass durch TMS insbesondere bei starker Stimulation im Gehirn des Patienten eine Krampfaktivität bzw. ein Krampfanfall ausgelöst werden kann. Es wird daher vorgeschlagen, das EEG des Patienten zu überwachen und falls darin eine Krampfaktivität erkennbar ist, die transkranielle Magnetstimulation abzuschalten. Ferner wird vorgeschlagen, die TMS zu beenden, wenn sich das Gehirn des Patienten basierend auf einer Auswertung der EEG Daten in einem gewünschten Ziel-Zustand befindet.

US 2010/0210894 A1 sowie WO 2008/070001 A2 aus der gleichen Patentfamilie offenbaren gemäß einem ersten Aspekt eine haubenartige Vorrichtung mit einer TMS-Spulenanordnung und in der Umgebung der Spulenordnung angeordneten Elektroden. In einer Notfallsituation soll diese Haube schnell über den Kopf eines Patienten gestülpt werden können, um einen Krampfanfall zu beenden. Gemäß einem zweiten Aspekt wird eine helmartige Positionierhilfe vorgeschlagen. Die Positionierhilfe kann EEG-Elektroden aufweisen. Ähnlich zur WO 2014/139029 A1 weist die Positionierhilfe Befestigungsmittel auf, an welchen eine TMS-Spulenanordnung mittels korrespondierender Befestigungsmittel ortsfest befestigt werden kann. Eine helmartige Positionierhilfe, um eine TMS-Spulenanordnung in einer vorgegebenen Position bezüglich des Kopfes des Nutzers zu halten ist auch aus der US 2010/0113959 A1 bekannt.

US 2002/0007128 A1 offenbart ferner, dass durch die TMS Pulse Wirbelströme in den metallischen EEG Elektroden induziert werden können. Dadurch können sich die Elektroden erhitzen. Es wird daher ferner die Verwendung von TMS kompatiblen Plastik-Elektroden bei ansonsten unveränderter Elektrodenanordnung vorgeschlagen.

WO 2014/140432 A1 offenbart ebenfalls die Überwachung eines EEGs eines Patienten während TMS aus Gründen der Patientensicherheit und zur Überwachung der Wirksamkeit. Da die TMS-Pulse das EEG System beeinflussen können, wird vorgeschlagen, EEG Daten dann zu messen, wenn gerade kein TMS-Puls anliegt, also beispielsweise unmittelbar nach einem TMS-Puls.

In Zrenner et al. "Brain-state dependent brain stimulation: Real-time EEG alpha band analysis using sliding window FFT phase progression extrapolation to trigger an alpha phase locked TMS pulse with 1 millisecond accuracy", First International Brain Stimulation Conference, 2. bis 4. März 2015, einer Veröffentlichung der Erfinder, wird beschrieben, dass ein Echtzeitelektroenzephalogramm zur hirnzustandsabhängigen Hirnstimulation verwendet werden kann. Dies wird auch als "EEG-TMS" bezeichnet. Zur Bestimmung des Hirnzustands wird ein konventionelles EEG in Echtzeit gemessen und die Aktivität der Alpha-Wellen bestimmt. Für die Positionierung der EEG Elektroden auf der Kopfhaut wird eine EEG-Haube verwendet, so dass die Elektroden an definierten Positionen angeordnet sind und vergleichbare Resultate erzielt werden können. Die Phasenlage der Alpha-Wellen wird mittels eines wandernden FFT-Fensters detektiert. Basierend auf der detektierten Phasenlage wird die transkranielle Magnetstimulation phasensynchron getriggert.

Nachteile der vorstehend gekannten Lösungen sind die hohe Komplexität der Systeme und die relativ komplizierte Handhabung einschließlich der langen Zeitdauer für die Inbetriebnahme, so dass deren Einsatz im klinischen Alltag in vielen Fällen nicht praktikabel erscheint. Ein Arzt könnte somit geneigt sein, eine medikamentöse Behandlung dem Einsatz eines TMS Systems vorzuziehen.

Vor diesem Hintergrund liegt der vorliegenden Erfindung die Aufgabe zugrunde, eine Vorrichtung zur transkraniellen Magnetstimulation derart weiterzubilden, dass vorzugsweise eine gezieltere und damit effektivere Stimulation ermöglicht und die Handhabung vereinfacht wird. Insbesondere soll die vorliegenden Vorrichtung die Applikation einer Stimulationsabfolge ermöglichen, welche nicht wie in herkömmlichen Protokollen vorprogrammiert und statisch ist, sondern dynamisch durch eine gleichzeitig vorliegende Hirnaktivität (durch eine EEG Ableitung gemessen) der individuellen Person gesteuert wird.

Gemäß einem ersten Aspekt der vorliegenden Erfindung wird eine Spulenanordnung mit einem Gehäuse und einer darin angeordneten Magnetspule zum Erzeugen eines Magnetfeldes zur transkraniellen Magnetstimulation vorgeschlagen, wobei die Spulenanordnung ferner eine an dem Gehäuse angeordnete Elektrodenanordnung zur Ableitung eines EEG-Signals aufweist, wobei die Magnetspule und die Elektrodenanordnung übereinander angeordnet sind, wobei die Elektrodenanordnung wenigstens eine feste Elektrode und wenigstens eine Federelektrode aufweist.

Es wird somit vorgeschlagen, eine Elektrodenanordnung zur Aufnahme eines EEG Signals direkt an dem Gehäuse der Spulenanordnung zur TMS vorzusehen. Die Elektrodenanordnung kann also bereits in der Spulenanordnung integriert sein. Ein wesentlicher Vorteil dieser Lösung besteht darin, dass die Messung der Hirnaktivität des Patienten bzw. Probanden direkt dort erfolgen kann, wo die Magnetspule platziert wird. Es besteht eine feste Positionsbeziehung zwischen TMS-Spule und EEG-Elektroden. Es kann also basierend auf dem EEG-Signal eine gezielte Stimulation genau an der Stelle der Messung der Hirnaktivität erfolgen, unabhängig davon, wo die Spulenanordnung platziert wird.

Es ist dabei anzumerken, dass bei konventioneller EEG-Signalerfassung üblicherweise sehr genau auf die Positionierung der Elektroden geachtet wird, um aussagekräftige, vergleichbare Messergebnisse bei verschiedenen Patienten zu erhalten. Bei der konventionellen Untersuchung mit dem EEG werden Elektroden an bestimmte Stellen der Schädeloberfläche angebracht, um die kortikale elektrische Aktivität zu messen. Das Schema der Positionierung hängt von der Anzahl der Elektroden ab. Üblicherweise kommt das internationale 10-20 System zum Einsatz. Es besteht also eine feste Positionsbeziehung zwischen dem Kopf des Probanden und den EEG-Elektroden. Als Positionierhilfe kann eine EEG-Haube mit einer Vielzahl von EEG-Elektroden an definierten Positionen verwendet werden. Dank der einheitlichen Positionierung können die Messergebnisse von verschiedenen Patienten miteinander verglichen werden, beispielsweise um charakteristische EEG-Muster für einen Krampfanfall zu erkennen.

Indem die Elektrodenanordnung fest am Gehäuse der Spulenanordnung angeordnet ist schlagen die Erfinder somit eine Abkehr von der üblichen Art der EEG Messung vor, wie sie im Stand der Technik eingesetzt wird. Die Erfinder haben erkannt, dass eine EEG basierte transkranielle Magnetstimulation (EEG-TMS) bereits basierend auf einem EEG-Signal, welches an der Position der Spulenanordnung aufgenommen wird, durchgeführt werden kann. Es ist also für EEG-TMS insbesondere nicht notwendig, stets ein EEG des gesamten Kopfes aufzunehmen.

Die vorgeschlagene Lösung bietet ferner den Vorteil, dass bei einer EEG basierten transkranielle Magnetstimulation eine EEG-Aktivität genau desjenigen Bereichs des Hirns berücksichtigt wird, welcher unmittelbar unter der Spulenanordnung liegt und vorzugsweise auch durch die TMS stimuliert wird. Hierzu sind die Magnetspule und die Elektrodenanordnung unmittelbar übereinander angeordnet. Insbesondere ist keine - potentiell fehlerbehaftete - Zuordnung einer oder mehrerer EEG Elektroden einer EEG Haube zu einer Position der Spulenanordnung erforderlich. Damit wird ferner die Komplexität des Systems reduziert und die Handhabung erleichtert.

Ein weiterer Vorteil der vorgeschlagen Lösung besteht darin, dass der Magnetpuls zur transkraniellen Magnetstimulation nicht durch weitere EEG-Elektroden, wie beispielsweise bei einer EEG-Haube, abgeschirmt wird. Ein weiterer Vorteil ist somit auch, dass sich keine weiteren EEG-Elektroden durch durch TMS-Magnetpulse induzierte Wirbelströme aufheizen können. Vorzugsweise ist die Elektrodenanordnung gemäß der vorgeschlagenen Lösung thermisch mit der Spulenanordnung gekoppelt, so dass Wärme von den EEG-Elektroden über die Spulenanordnung abgeführt werden kann. Demgegenüber wird im Stand er Technik (US 2002/0007128 A1) der Einsatz von Polymer-Elektroden vorgeschlagen.

Weitere Vorteile bestehen in einer vereinfachten Handhabung und Zeitersparnis im Betrieb, da es nicht erforderlich ist vor der eigentlichen TMS Prozedur zunächst die EEG-Elektroden auf der Kopfhaut des Patienten anzubringen. Mit der korrekten Positionierung von EEG-Elektroden ist üblicherweise ein hoher Zeitaufwand von beispielsweise 30 min verbunden. Darüber hinaus können mit der vorgeschlagenen Lösung die Kosten gegenüber separaten EEG- und TMS-Systemen reduziert werden. Vorzugsweise kann eine therapeutische Applikation beispielsweise eines 20-Minuten-Protokolls 3 Mal am Tag ermöglicht werden.

Ein weiterer Vorteil besteht darin, dass die EEG-Messung unterhalb der Stelle erfolgen kann, von welcher die nachfolgende transkranielle Magnetstimulation durch die Magnetspule ausgeht. Ein weiterer Vorteil ist ein kompakter Aufbau.

Vorzugsweise ist die Elektrodenanordnung bezüglich der Magnetspule zentriert angeordnet.

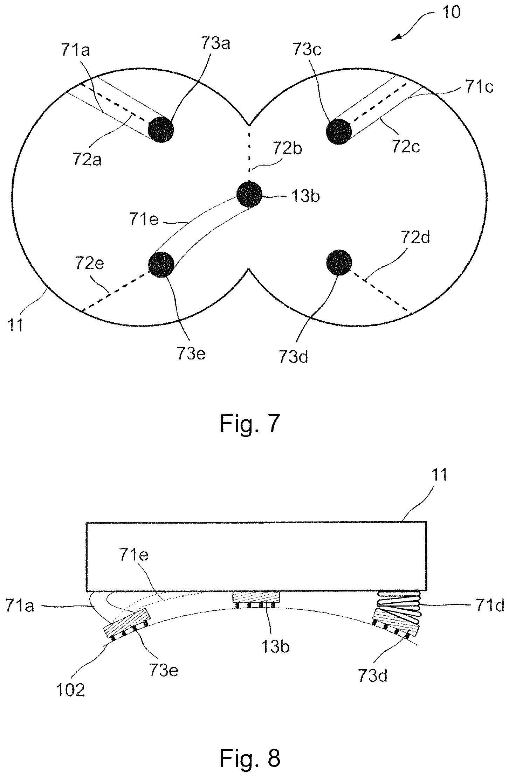

Ein Vorteil dieser Ausgestaltung ist, dass die Positionierung und damit die Handhabung der Elektrodenanordnung erleichtert wird. Die Lage der Magnetspule ist vorzugsweise auf einer Oberseite der Spulenanordnung gekennzeichnet. Wenn die Spulenanordnung mit der Elektrodenanordnung an einer Unterseite der Spulenanordnung auf dem Kopf des Patienten platziert wird, kann sich der Bediener, z.B. Arzt, also an der Lage der Magnetspule orientieren. Die Elektrodenanordnung und die Magnetspule können also in axialer Richtung der Magnetspule aneinander ausgerichtet und zentriert sein. Die Elektrodenanordnung kann insbesondere derart angeordnet sein, dass die Elektroden der Elektrodenanordnung ein Zentrum der Magnetspule umgeben. Ein Zentrum der Magnetspule kann sich bei einer ringförmigen Spule auf die Mitte des Rings beziehen. Ein Zentrum der Magnetspule kann sich bei einer achterförmigen Spule (Figure8-Coil) auf die Mitte der Acht, also auf den Schnittpunkt der linken und rechten Windungsbereiche, oder alternativ auf ein Zentrum des rechten oder linken Windungsbereichs beziehen.

Vorzugsweise weist die Spulenanordnung genau eine Magnetspule zum Erzeugen eines Magnetfeldes zur transkraniellen Magnetstimulation auf.

Die Spule kann als ringförmige Spule oder achterförmige Spule bzw. Spule mit einer achterförmigen Windung ausgebildet sein. Die Spule weist vorzugweise einen Durchmesser zwischen 30mm und 150mm, vorzugsweise zwischen 40mm und 120mm, vorzugsweise zwischen 50mm und 90mm auf.

Vorzugsweise weist das Gehäuse ferner einen Handgriff oder eine Haltevorrichtung auf.

Mit dem Handgriff oder einer alternativen Haltevorrichtung kann ein Bediener, z.B. Arzt, die Spulenanordnung einschließlich der Elektrodenanordnung frei entlang des Kopfes des Patienten bewegen und so die gewünschte Stelle zur TMS auswählen. Ferner kann eine Haltevorrichtung derart ausgebildet sein, dass die Spulenanordnung an einem Halter befestigt werden kann. Dies ist vorteilhaft, wenn über eine längere Zeit die gleiche Stelle stimuliert werden soll.

Vorzugsweise weist die Elektrodenanordnung wenigstens eine Trockenelektrode oder eine Nasselektrode, insbesondere eine Elektrode aus einem superabsorbierenden Polymer auf.

Für TMS-kompatible EEG-Messungen gemäß dem Stand der Technik werden üblicherweise Gel-Elektroden eingesetzt. Bei der vorgeschlagenen Elektrodenanordnung werden jedoch vorzugweise die vorstehenden genannten Trockenelektroden oder Elektroden mit einem superabsorbierenden Polymer eingesetzt. Eine derartige Elektrode mit superabsorbierenden Polymer ist aus der DE 10 2012 101 337 A1 bekannt. Diese Elektroden werden auch als "wet marble electrodes" bezeichnet. Trockenelektroden sind beispielsweise kommerziell verfügbar von der Firma Cognionics Inc., USA. Ein Vorteil der vorstehend genannten Elektroden gegenüber Gel-Elektroden besteht darin, dass die Spulenanordnung auf dem Kopf des Patienten leichter verschoben werden kann.

Die Elektrodenanordnung wenigstens eine feste Elektrode und wenigstens eine Federelektrode auf.

Ein Vorteil von Federelektroden besteht darin, dass sie sich flexibel der Kopfform des Nutzers anpassen können. Es kann somit ein besserer Kontakt und damit ein besseres EEG-Signal erreicht werden. Eine oder mehrere Elektroden der Elektrodenanordnung können als Federelektroden ausgebildet sein. Federelektroden werden auch als 'spring-loaded electrodes' bezeichnet. Federelektroden weisen jedoch üblicherweise eine höhere Bauhöhe auf. Vorzugsweise ist daher wenigstens eine Elektrode der Elektrodenanordnung keine Federelektrode sondern eine feste bzw. starre Elektrode. Die Erfinder haben erkannt, dass für die vorliegende Anwendung eine Kombination aus wenigstens einer Federelektrode und wenigstens einer festen Elektrode besonders vorteilhaft sein kann. Durch die feste Elektrode kann ein geringerer Abstand zwischen dem Kopf des Nutzers und der Magnetspule erreicht werden. Ein geringer Abstand kann zu einer besseren transkraniellen Magnetstimulation durch das von der Magnetspule erzeugte Magnetfeld führen. Durch die Kombination einer festen Elektrode mit Federelektroden kann jedoch gleichzeitig eine flexible Anpassung an die Kopfform des Nutzers erfolgen. Dadurch können die Wirkung des Magnetfeldes, die EEG-Signalqualität und/oder die Handhabung der Spulenanordnung dank der flexibleren Anpassung verbessert werden.

Ein weiterer Vorteil besteht darin, dass die Elektrodenanordnung schnell und einfach auf dem Schädel des Nutzers positioniert werden kann. Dies ist insbesondere vorteilhaft, wenn die TMS-Spulenanordnung frei positioniert wird, beispielsweise um flexibel unterschiedliche Hirnareale stimulieren zu können.

In einer Weiterbildung kann die Elektrodenanordnung wenigstens eine feste Elektrode und eine Mehrzahl an Federelektroden aufweisen, wobei die Federelektroden die wenigstens eine feste Elektrode umgeben.

Beispielsweise können eine, zwei oder drei feste Elektroden vorgesehen sein, welche von weiteren Federelektroden umgeben sind. Die Federelektroden in der Umgebung können sich flexibel der Kopfform des Nutzers anpassen.

In einer Weiterbildung kann die Spulenanordnung die wenigstens eine feste Elektrode bezüglich der Magnetspule zentriert angeordnet sein.

Mit anderen Worten kann die wenigstens eine feste Elektrode in einem zentralen Bereich der Magnetspule (unterhalb der Magnetspule am Gehäuse) angeordnet sein. Ein Vorteil dieser Ausgestaltung besteht insbesondere darin, dass in einem zentralen Bereich durch die feste Elektrode ein geringer Abstand zwischen Magnetspule und Kopf erreicht werden kann. Insbesondere bei einer achterförmigen Spule bildet sich das Feldmaximum in einem zentralen Bereich der Magnetspule aus. Somit kann eine verbesserte Ankopplung und verbesserte transkranielle Magnetstimulation im Zentrum erreicht werden.

Vorzugsweise weist die Spulenanordnung ferner eine integrierte Auswerteeinheit zur Auswertung des EEG-Signals auf. Alternativ kann die Auswerteeinheit auch in einem anderen Teil eines EEG-basierten TMS-Systems angeordnet sein. Hierfür kann die Auswerteeinheit beispielsweise über ein Kabel mit der Spulenanordnung verbunden sein. Die Auswerteeinheit kann in die Elektrodenanordnung integriert sein.

Ein Vorteil dieser Ausgestaltung besteht insbesondere darin, dass an anderen Komponenten eines TMS-Systems, insbesondere dem Stimulusgenerator, keine oder nur minimale Änderungen erforderlich sein können. Vorzugsweise kann ein konventioneller Stimulusgenerator mit einem Triggereingang ohne weitere Modifikationen eingesetzt werden. Die Auswerteeinheit kann dazu ausgebildet sein, basierend auf einer Auswertung des EEG-Signals ein Triggersignal zu erzeugen, welches an einen Triggereingang des Stimulusgenerators übermittelt wird.

Ein weiterer Vorteil besteht darin, dass das EEG-Signal direkt in der Spulenanordnung verarbeitet werden kann. Es ist also nicht erforderlich, EEG-Rohsignale zu weiteren Einheiten zu leiten.

In einer Weiterbildung kann die Auswerteeinheit dazu ausgebildet sein, um basierend auf dem EEG-Signal eine Anpassung eines Parameters der transkraniellen Magnetstimulation zu bewirken.

Ein Vorteil dieser Ausgestaltung besteht darin, dass die transkranielle Magnetstimulation somit unmittelbar auf den durch das EEG-Signal repräsentierten individuellen Hirnzustand angepasst werden kann. Insbesondere kann als Parameter ein Zeitpunkt oder eine Intensität, Leistung bzw. Pulsenergie, eine Dauer und/oder Frequenz von Magnetstimuli der TMS angepasst werden. Beispielsweise kann die Phasenlage einer Alpha-Welle des Hirns bestimmt und der TMS-Puls basierend auf der Phasenlage der Alpha-Welle ausgelöst werden, indem die Auswerteeinheit ein entsprechendes Triggersignal an einen Stimulusgenerator übermittelt. Vorzugsweise erfolgt die Auswertung des EEG-Signals in einem Echtzeit-Datenverarbeitungssystem (z. B. ein LabView oder Simulink Real-Time Modell auf einer FPGA Plattform).

Die Bestimmung der augenblicklichen (d.h. zum Zeitpunkt einer möglichen Stimulation vorliegenden) Phasenlage des EEG Signals in einem bestimmten Frequenzband ist nicht trivial, da das in Echtzeit verarbeitete und gefilterte Signal notwendigerweise im Vergleich zu dem ursprünglichen Signal verzögert ist (typischerweise um ca. 100 Millisekunden, d.h. eine komplette Alpha-Welle). In einer Weiterbildung kann die Auswerteeinheit dazu ausgebildet sein, die augenblickliche Phasenlage durch einen oder mehrere Ansätze in Kombination zu bestimmen.

Ein erster von den Erfindern in dem vorliegenden Prototypen implementierter Ansatz ist die dynamische DFT (diskrete Fouriertransformation) eines unmittelbar vorausgehenden EEG Signals: In einer Weiterbildung kann die die Auswerteeinheit daher dazu ausgebildet sein,
eine, insbesondere jeweils augenblickliche, Phasenlage des EEG-Signals in einem vorgegebenen Frequenzband zu bestimmen,
wobei in einem ersten Schritt eine Bestimmung einer ersten Frequenz des EEG-Signals mittels einer ersten Spektralanalyse eines ersten gleitenden Fensters des EEG-Signals erfolgt,
und in einem zweiten Schritt eine Phase des EEG-Signals mittels einer zweiten Fourieranalyse eines zweiten gleitenden Fensters des EEG-Signals erfolgt,
wobei das erste Fenster vorzugsweise breiter ist als das zweite Fenster,
und wobei die Breite des zweiten Fensters für die zweite Spektralanalyse derart gewählt ist, dass sie einem ganzzahligen Vielfachen einer Wellenlänge entsprechend der ersten Frequenz des EEG-Signals entspricht,
oder alternativ eine DFT Gleichung mit einem nicht-ganzzahligen, an die Wellenlänge entsprechend der ersten Frequenz des EEG-Signals angepasstem Multiplikationsparameter ermittelt wird.

Ein Vorteil dieser Ausgestaltung ist, dass die augenblickliche Phasenlage des EEG-Signals soweit möglich exakt bestimmt werden kann. Der Einfluss von Artefakten kann reduziert werden. Mit anderen Worten wird ein zweistufiges Vorgehen vorgeschlagen. In dem ersten Schritt erfolgt eine grobe Bestimmung einer gewünschten ersten Frequenz des EEG Signals. Beispielsweise kann es sich bei der gewünschten ersten Frequenz um eine Alpha-Welle handeln. Als Alpha-Wellen wird bei EEG-Messungen ein Signal im Frequenzbereich zwischen 8 und 13 Hz bezeichnet. In dem ersten Schritt kann somit die erste Frequenz in einem gewünschten Frequenzbereich ermittelt werden, beispielsweise die Frequenz mit der höchsten Amplitude in diesem Frequenzbereich. In dem zweiten Schritt wird basierend auf der bestimmten ersten Frequenz die Breite des zweiten Fensters für die Fourieranalyse derart gewählt, dass sie einem ganzzahligen Vielfachen der Wellenlänge bei der bestimmten ersten Frequenz entspricht. Es wird also eine Form kaskadierter Auswertung vorgeschlagen.

Ein zweiter von den Erfindern in einem vorliegenden Prototypen implementierter Ansatz ist ein autoregressives Echtzeit-Vorwärtsmodell, welches die durch die Filterung verursachte Verzögerung des Signals durch eine Vorausberechnung des erwarteten Signals basierend auf das zurückliegende Signal erstellt und die Phase des vorausberechneten Signals analysiert. Ein solcher Ansatz wurde vorgestellt in Chen LL, Madhavan R, Rapoport BI, Anderson WS, Real-Time Brain Oscillation Detection and Phase-Locked Stimulation Using Autoregressive Spectral Estimation and Time-Series Forward Prediction, IEEE transactions on bio-medical engineering, 2013;60(3):753-762.

Vorzugsweise weist die Spulenanordnung eine Schutzschaltung zur Abschaltung von wenigstens einer Komponente zur Messung des EEG-Signals während eines Magnetpulses auf.

Ein Vorteil dieser Ausgestaltung kann darin besehen, dass eine Beschädigung von Komponenten zur Messung des EEG-Signals während eines Magnetpulses vermieden werden können. Vorzugsweise stehen die Komponenten zur Messung des EEG-Signals ohne Beeinträchtigung durch einen TMS-Puls schneller wieder für eine nachfolgende EEG-Messung bereit.

Vorzugsweise erfolgt nach Erzeugen des Magnetfeldes ein Reset wenigstens einer Komponente zur Messung und/oder Verarbeitung des EEG-Signals und/oder zur Steuerung eines Parameters zur transkraniellen Magnetstimulation.

Ein Magnetpuls bzw. durch die Magnetspule erzeugtes Magnetfeld zur transkraniellen Magnetstimulation kann die Funktionalität von Komponenten zur Messung des EEG-Signals beeinträchtigen. Vorzugsweise wird nach einem derartigen Magnetpuls ein Reset bzw. Neustart wenigstens einer Komponente zur Messung des EEG-Signals und/oder zur Verarbeitung des EEG-Signals und Steuerung eines Stimulationsparameters zur transkraniellen Magnetstimulation durchgeführt. Beispielsweise erfolgt eine erneute Initialisierung von Analog-zu-Digital-Wandlern zur Digitalisierung eines rohen EEG-Signals. Ein Vorteil von dieser wie auch der vorigen Ausführung besteht darin, dass Artefakte vermieden werden können und/oder eine Steuerelektronik gegenüber Interferenzen durch den Magnetpuls robust gemacht wird.

Vorzugsweise weist die Spulenanordnung ferner eine Referenzelektrode auf.

Ein Vorteil dieser Ausgestaltung besteht darin, dass mittels der Referenzelektrode ein Bezugspotential für das EEG-Signal bereitgestellt werden kann. Die Referenzelektrode kann beispielsweise ausgebildet sein zur Befestigung an einem im Wesentlichen elektrisch neutralen Ort, wie einem Ohrläppchen oder einem Schlüsselbein. Die Referenzelektrode kann über ein Kabel mit der Spulenanordnung verbunden sein.

Ferner kann die Spulenanordnung eine oder mehrere zusätzliche über ein Kabel konnektierte fixierte EEG Elektroden aufweisen (z. B. auch Gel-Elektroden, die aufgeklebt werden, oder ein Netz, oder z. B. auch Elektroden, die in ein Stirnband eingebracht sind), z. B. um den Effekt der Stimulation auf evozierte EEG-Potentiale in von der Stimulationselektrode weiter entfernten Hirnarealen als Effekt-Parameter zu messen.

Vorzugsweise weist die Spulenanordnung eine Elektrode zur Ableitung eines EMG-Signals auf.

Ein Vorteil dieser Ausgestaltung besteht darin, dass die vorgeschlagene Vorrichtung somit die Applikation einer transkranialen Stimulationsabfolge ermöglicht, welche eine über ein EMG (Elektromyogramm) gemessene Muskelaktivität der individuellen Person gesteuert wird. Vorzugsweise kann die Auswerteeinheit dazu ausgebildet sein, um basierend auf dem EMG-Signal eine Anpassung eines Parameters der transkraniellen Magnetstimulation zu bewirken. Es kann also eine hirnzustandsabhängige Hirnstimulation basierend auf dem EEG-Signal und/oder dem EMG-Signal erfolgen. Die Spulenanordnung kann eine oder mehrere, auch zusätzliche, insbesondere über ein Kabel konnektierte sonstige Biosignal-Elektroden aufweisen, z. B. EMG-Ableitungen um den Effekt der Stimulation auf evozierte Muskelpotentiale als Effekt-Parameter zu messen.

Gemäß einem zweiten Aspekt der vorliegenden Erfindung wird eine Elektroden-anordnung zur Aufnahme bzw. Ableitung eines EEG-Signals vorgeschlagen, wobei die Elektrodenanordnung einen Halter aufweist, der an das Gehäuse einer Spulenanordnung angepasst ist, wobei die Spulenanordnung eine in dem Gehäuse angeordnete Magnetspule zum Erzeugen eines Magnetfeldes zur transkraniellen Magnetstimulation aufweist, und wobei die Elektrodenanordnung mittels des Halters an dem Gehäuse der Spulenanordnung anbringbar ist, derart dass die Magnetspule und die Elektrodenanordnung übereinander angeordnet sind, wobei die Elektrodenanordnung wenigstens eine feste Elektrode und wenigstens eine Federelektrode aufweist.

Die vorstehende genannte Lösung ermöglicht somit die nachträgliche Ausstattung einer TMS-Spulenanordnung bzw. eines TMS-Systems mit der EEG-Funktionalität wie ausführlich gemäß dem ersten Aspekt der vorliegenden Erfindung beschrieben. Mit anderen Worten kann eine bestehende Spulenanordnung nachträglich mit der EEG-Funktionalität ausgestattet werden. Die vorgeschlagene Elektrodenanordnung mit Halter kann somit als eine Art Adapter bzw. Elektrodenaufsatz für eine bestehende TMS-Spulenanordnung verstanden werden. In Verbindung mit einer Spulenanordnung ermöglicht der vorgeschlagene Elektrodenadapter somit die gleichen Vorteile wie die Spulenanordnung mit Elektrodenanordnung gemäß dem ersten Aspekt der vorliegenden Erfindung.

Vorzugsweise weist die Elektrodenanordnung eine integrierte Auswerteeinheit zum Auswerten des EEG-Signals und optional zum Erzeugen eines Triggersignals auf. Im Allgemeinen weist die Elektrodenanordnung eine Auswerteeinheit auf. Die Auswerteeinheit kann alternativ auch über ein Kabel an die Elektrodenanordnung angeschlossen sein. Die Auswerteeinheit kann zum Modulieren eines Parameters eines Algorithmus, mit dem ein Triggersignal erzeugt wird, ausgebildet sein.

Ein Vorteil dieser Ausgestaltung besteht insbesondere darin, dass an anderen Komponenten eines TMS-Systems, insbesondere an der Spulenanordnung und dem Stimulusgenerator, keine oder nur minimale Änderungen erforderlich sein können. Vorzugsweise kann ein konventioneller Stimulusgenerator mit einem Triggereingang ohne weitere Modifikationen eingesetzt werden. Die Auswerteeinheit kann dazu ausgebildet sein, basierend auf einer Auswertung des EEG-Signals ein Triggersignal zu erzeugen, welches an einen Triggereingang des Stimulusgenerators übermittelt wird.

Gemäß einem dritten Aspekt der vorliegenden Erfindung wird ein System zur transkraniellen Magnetstimulation bereitgestellt, wobei das System Folgendes aufweist:
- eine vorstehend bereits beschriebene Spulenanordnung gemäß dem ersten Aspekt der Erfindung oder eine Spulenanordnung sowie eine Elektrodenanordnung gemäß dem zweiten Aspekt der Erfindung;
- einem Stimulusgenerator zum Erzeugen eines transkraniellen magnetischen Stimulus; und
- einer Steuereinheit zum Ansteuern des Stimulusgenerators.

Mit anderen Worten kann das System einerseits eine Spulenanordnung mit einer bereits an dem Gehäuse angeordneten bzw. darin integrierten Elektrodenanordnung aufweisen. Andererseits kann das System eine konventionelle Spulenanordnung aufweisen und zusätzlich die Elektrodenanordnung zur Aufnahme eines EEG-Signals, welche mittels des Halters an dem Gehäuse der Spulenanordnung anbringbar ist. Vorzugsweise sind an dem Gehäuse der Spulenanordnung bereits Mittel zum Befestigen des Halters vorgesehen, wie beispielsweise Ausnehmungen im Gehäuse der Spulenanordnung zum Eingriff von Schnapphaken des Halters der Elektrodenanordnung.

Der Stimulusgenerator ist mit der Spulenanordnung verbunden und kann, indem auf bekannte Art und Weise Strom durch die Magnetspule der Spulenanordnung geleitet wird, einen transkraniellen magnetischen Stimulus erzeugen. Mit anderen Worten wird durch einen vom Stimulusgenerator bereitgestellten Strom ein Magnetpuls durch die Magnetspule erzeugt, welcher eine transkranielle magnetische Stimulation bewirken kann. Ein derartiger Magnetpuls wird daher in der vorliegenden Offenbarung als transkranieller magnetischer Stimulus bezeichnet.

Es versteht sich, dass die vorstehend genannte Elektrodenanordnung und das vorstehend genannte System gemäß dem zweiten und dritten Aspekt der Erfindung identische oder ähnliche Ausgestaltungen und/oder Vorteile wie die Spulenanordnung gemäß dem ersten Aspekt der E aufweisen können. Elemente, welche in der Spulenanordnung vorgesehen sind, können alternativ auch in der Elektrodenanordnung oder im System, beispielsweise in der Steuereinheit des Systems, vorgesehen sein. So kann beispielsweise die Auswerteeinheit in der Spulenanordnung, in der Elektrodenanordnung oder in der Steuereinheit angeordnet sein.

Weitere Vorteile ergeben sich aus der Beschreibung und den beigefügten Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den beigefügten Zeichnungen dargestellt und werden in der nachfolgenden Beschreibung näher erläutert. Es zeigen:
Figur 1 eine schematische Darstellung eines Systems zur transkraniellen Magnetstimulation (TMS) gemäß einem Ausführungsbeispiel der Offenbarung;
Figur 2 eine Unteransicht einer Spulenanordnung zur transkraniellen Magnetstimulation mit einer integrierten Elektrodenanordnung gemäß einem Ausführungsbeispiel der Offenbarung;
Figur 3 eine Draufsicht einer Spulenanordnung mit einer Elektrodenanordnung in Form eines Adapters gemäß einem Ausführungsbeispiel der Offenbarung;
Figur 4 eine Unteransicht der Spulenanordnung mit der Elektrodenanordnung in Form eines Adapters gemäß Fig. 3;
Figur 5 eine schematische Darstellung einer Auswerteeinheit gemäß einem Ausführungsbeispiel der Offenbarung;
Figur 6 ein Flussdiagramm zur Auswertung eines EEG-Signals gemäß einem Ausführungsbeispiel der Offenbarung;
Figur 7 eine Unteransicht einer Spulenanordnung zur transkraniellen Magnetstimulation mit einer integrierten Elektrodenanordnung mit wenigstens einer festen Elektrode und einer Mehrzahl an Federelektroden gemäß einem Ausführungsbeispiel der Offenbarung; und
Figur 8 eine Seitenansicht der Spulenanordnung gemäß Figur 7.

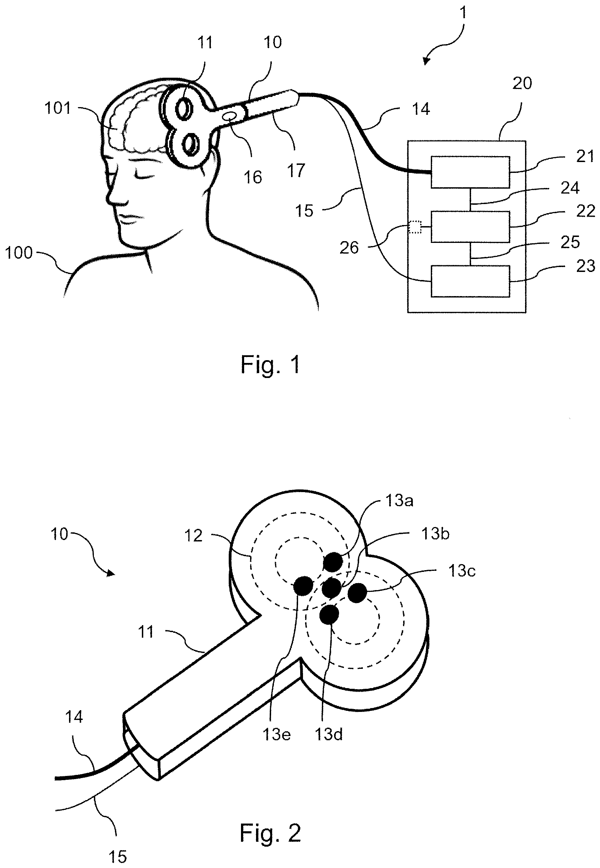

Fig. 1 zeigt eine schematische Darstellung eines Systems zur transkraniellen Magnetstimulation (TMS) des Gehirns 101 eines Patienten 100 gemäß einem Ausführungsbeispiel der Offenbarung. Das System ist in seiner Gesamtheit mit Bezugszeichen 1 bezeichnet.

In Fig. 2 ist eine Unteransicht einer Spulenanordnung 10 zur transkraniellen Magnetstimulation aus Fig. 1 gezeigt. Auf der Unterseite weist die Spulenanordnung 10 eine integrierte Elektrodenanordnung 13 auf, welche beispielhaft durch die Elektroden 13a bis 13e dargestellt ist.

Das System 1 zur transkraniellen Magnetstimulation weist in diesem Ausführungsbeispiel eine Spulenanordnung 10 und eine Stimuluseinheit 20 auf. Die Stimuluseinheit 20 weist einen Stimulusgenerator 21 zum Erzeugen eines transkraniellen magnetischen Stimulus, eine Steuereinheit 22 zum Ansteuern des Stimulusgenerators 21, und eine Auswerteeinheit 23 auf. Die Auswerteeinheit 23 kann auch beispielsweise in der Spulenanordnung 10 angeordnet sein.

Die Spulenanordnung 10 weist ein Gehäuse 11 und eine darin angeordnete Magnetspule 12 zum Erzeugen eines Magnetfeldes zur transkraniellen Magnetstimulation auf, wobei die Spulenanordnung 10 ferner eine an dem Gehäuse 11 angeordnete Elektrodenanordnung 13 zur Aufnahme eines EEG-Signals aufweist. Die Magnetspule 12 in dem Gehäuse 11 ist in Fig. 2 durch eine gestrichelte achterförmige Spulenkonfiguration dargestellt. Alternativ können andere Spulenkonfigurationen bzw. eine andere Spulengeometrie wie beispielsweise eine Ringspule eingesetzt werden.

Um die TMS am Gehirn 101 eines Patienten 100 durchzuführen, kann die Spulenanordnung 10 zunächst mit den Elektroden 13a-13e in Kontakt mit der Kopfhaut des Patienten 100 gebracht werden. Das Gehäuse 11 der Spulenanordnung kann hierfür einen Handgriff 17 aufweisen, mit welchem die Spulenanordnung 10 von einem Bediener, wie beispielsweise einem Arzt, gehalten werden kann. Mittels der Elektroden 13a-13e kann ein EEG-Signal des Patienten 100 aufgenommen werden. Das EEG-Signal bzw. eine Mehrzahl von EEG-Signalen können über eine Signalleitung 15 an die Auswerteeinheit 23 übertragen werden. Alternativ kann die Signalübertragung auch kabellos erfolgen.

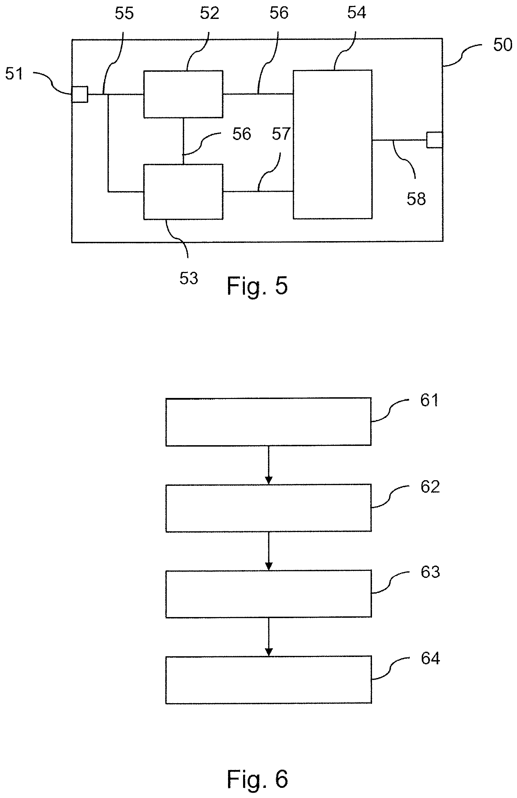

Die Auswerteeinheit 23 wertet das EEG-Signal aus und erzeugt basierend auf dem EEG-Signal ein Triggersignal 25, welches an die Steuereinheit 22 übertragen wird. Vorzugsweise kann die Auswerteeinheit eine Phasenlage beispielsweise der Alpha-Wellen des EEG-Signals in Echtzeit bestimmen und ein entsprechendes Triggersignal erzeugen. Eine vorteilhafte Ausgestaltung einer Auswerteeinheit wird weiter unten unter Bezugnahme auf Fig. 5 und 6 erläutert.

Die Steuereinheit 22 erzeugt als Reaktion auf das Triggersignal 25 ein Ansteuersignal 24 zum Ansteuern des Stimulusgenerators 21. Der Stimulusgenerator 21 generiert als Reaktion auf das Ansteuersignal 24 einen starken sich zeitlich schnell verändernden Strom, welcher über die Stromleitung 14 durch die Magnetspule 12 in der Spulenanordnung 10 geleitet wird. Wie eingangs beschrieben wird dadurch ein Magnetfeld bzw. ein Magnetpuls erzeugt, welches einen Strom im Gehirn 101 des Patienten 100 induziert.

Durch diese Stimulation kann der Zustand des Gehirns 101 des Patienten 100 vorteilhaft beeinflusst werden. Der Zustand des Gehirns kann nach einem derartigen Stimulus erneut mittels der EEG-Elektroden 13 und der Auswerteeinheit 23 ausgewertet und ein erneuter Stimulus ausgelöst werden. Vorzugsweise entsteht so ein geschlossener Regelkreis zur EEG-basierten transkraniellen Magnetstimulation (EEG-TMS).

Indem die Elektroden 13a-13e unmittelbar am Gehäuse 11 der Spulenanordnung 10 angebracht sind, erfolgt die Stimulation stets an genau der Stelle, an welcher das zugrundeliegende EEG-Signal gemessen wurde. Es kann somit auf einfache Weise eine gezielte TMS erfolgen.

Optional kann die Spulenanordnung ferner einen Triggerschalter 16 zum manuellen Auslösen eines Magnetpulses oder einer TMS Sequenz aufweisen. Die Stromleitung 14 und die Signalleitung 15 sind beispielhaft als unterschiedliche Leitungen dargestellt, können aber auch in einem Kabel geführt werden, was die Handhabung vereinfacht. Optional weist die Auswerteeinheit 23 eine Schutzschaltung zur Abschaltung von wenigstens einer Komponente zur Messung des EEG-Signals während eines Magnetpulses auf.

Die Elektroden 13a-13e sind beispielsweise als Trockenelektroden ausgeführt, damit die Spulenanordnung 10 auf dem Kopf des Patienten 100 verschoben werden und so eine EEG-Messung und entsprechende Stimulation unterschiedlicher Bereiche des Gehirns erfolgen kann.

Wie in Fig. 2 gezeigt sind die Magnetspule 12 und die Elektrodenanordnung 13 übereinander angeordnet. Insbesondere sind die Elektroden 13a, 13c, 13d, 13e in einer beispielhaften Konfiguration um ein Zentrum der Magnetspule 12 herum angeordnet. Die EEG-Messung kann somit zentriert dort erfolgen, wo die Magnetspule 12 stimuliert. Die Elektrodenanordnung 13 weist ferner eine zentrale Elektrode 13b auf. Mit anderen Worten kann die EEG-Messung somit mit einer bezüglich der Magnetspule 12 ortsfesten Elektrodenanordnung erfolgen, welche insbesondere unmittelbar unterhalb der Magnetspule 12 angeordnet ist. Durch die zentrale Anordnung kann insbesondere bei Einsatz einer achterförmigen Magnetspule 12 eine EEG-Messung nahe des Feldmaximums erfolgen. Es versteht sich, dass die Elektrodenanordnung auch eine Mehrzahl zentral angeordneter Elektroden bzw. um ein Zentrum der Magnetspule 12 angeordnete Elektroden aufweisen kann.

Die Steuereinheit 22 kann vorzugsweise einen externen Triggereingang 26 aufweisen. Wenn beispielsweise die Auswerteeinheit 23 anstatt in der Stimuluseinheit 20 in der Spulenanordnung 10 angeordnet ist, kann die Auswerteeinheit über die Steuerleitung 15, welche dann mit dem Triggereingang 26 verbunden sein kann, ein Triggersignal an die Steuereinheit 22 der Stimuluseinheit 20 übertragen und so einen Magnetstimulus auslösen.

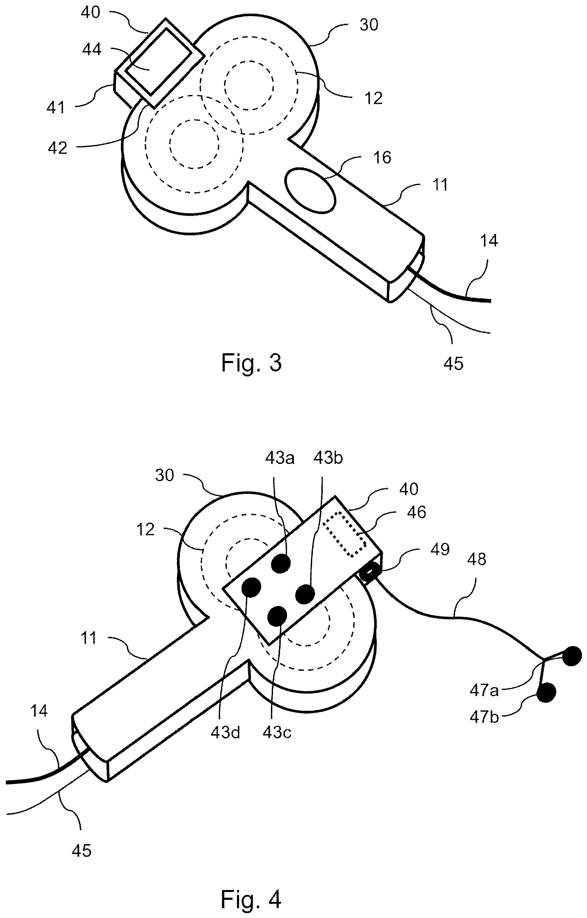

Fig. 3 zeigt eine Draufsicht einer Spulenanordnung 30 mit einer Elektrodenanordnung 40 in Form eines Adapters gemäß einem Ausführungsbeispiel der Offenbarung. Fig. 4 zeigt eine Unteransicht der Spulenanordnung 30 mit der Elektrodenanordnung 40 in Form eines Adapters gemäß Fig. 3.

Die Elektrodenanordnung 40 zur Aufnahme eines EEG-Signals weist einen Halter 41 auf, der an das Gehäuse 11 einer Spulenanordnung 30 angepasst ist, wobei die Spulenanordnung 30 eine in dem Gehäuse 11 angeordnete Magnetspule 12 zum Erzeugen eines Magnetfeldes zur transkraniellen Magnetstimulation aufweist, und wobei die Elektrodenanordnung 40 mittels des Halters 41 an dem Gehäuse 11 der Spulenanordnung 10 angebracht ist.

Als Alternative zu dem in Fig. 1 und Fig. 2 gezeigten Beispiel, bei welchem die Elektrodenanordnung 13 einen Bestandteil der Spulenanordnung 10 bildet, kann die Elektrodenanordnung 40 gemäß Fig. 3 und Fig. 4 somit auch nachträglich an eine bereits bestehende Spulenanordnung 30 angebracht werden. Derartige Spulenanordnungen 30 ohne EEG Elektroden sind aus dem Stand der Technik bekannt und kommerziell verfügbar.

Der Halter 41 kann beispielsweise ein U-förmiges Profil aufweisen, welches auf eine Vorderseite der Spulenanordnung 30 aufgesteckt werden kann. Eine Oberseite des Halters 41 kann damit eine Oberseite der Spulenanordnung überragen 42. Eine Unterseite des Halters 41 weist eine Mehrzahl von EEG-Elektroden 43a-43d auf und überragt eine Unterseite der Spulenanordnung 30. Die Spulenanordnung 30 kann somit in den Halter 41 der Elektrodenanordnung 40 eingeführt, vorzugsweise eingeklemmt werden. Die Elektrodenanordnung 40 wird somit an der Spulenanordnung 30 befestigt.

Die Elektrodenanordnung 40 kann optional eine Benutzerschnittstelle (HMI, Human-Machine-Interface) aufweisen. Ein Vorteil dieser Ausgestaltung ist, dass einem Bediener unmittelbar eine Rückmeldung zu dem gemessenen EEG-Signal gegeben werden kann. Basierend auf dieser Rückmeldung kann der Bediener beispielsweise die Positionierung der Spulenanordnung 30 und der Elektrodenanordnung 40 überprüfen und gegebenenfalls anpassen.

Vorzugsweise weist die Elektrodenanordnung 40, wie in Fig. 4 angedeutet, eine integrierte Auswerteeinheit 26 auf. Die Auswerteeinheit kann mittels der EEG-Elektroden 43a-43d erfasste EEG-Signale auswerten und basierend auf der Auswertung ein Triggersignal erzeugen.

Wie in Fig. 3 und Fig. 4 gezeigt weist die Spulenanordnung 30 ferner eine Stromleitung 14 auf, welche mit einem Stimulusgenerator 21 einer Stimuluseinheit 20 entsprechend Fig. 1 verbunden werden kann. Die Elektrodenanordnung 40 weist eine Steuerleitung 45 auf, welche mit einem Triggereingang 26 einer Steuereinheit 24 einer Stimuluseinheit entsprechend Fig. 1 verbunden werden kann.

Optional weist die Elektrodenanordnung 40 ferner wenigstens eine Referenzelektrode 47a, 47b auf, welche an einem im Wesentlichen elektrisch neutralen Ort des Körpers des Probanden positioniert werden und als Referenz für die EEG-Messung dienen kann. Die Referenzelektrode 47a bzw. 47b kann mittels eines Kabels 48 mit einem Eingang 49 der Elektrodenanordnung 40 verbunden werden.

Optional weist die Elektrodenanordnung 40 wenigstens eine EMG-Elektrode, also eine Elektrode zur Aufnahme eines Elektromyogramms auf. Diese kann entsprechend als EMG-Elektrode 47a, 47b, wie in Fig. 4 ausgeführt sein. Die EMG-Elektrode kann mittels eines Kabels 48 mit einem Eingang 49 der Elektrodenanordnung verbunden werden. Es versteht sich, dass sowohl eine EMG-Elektrode als auch eine Referenzelektrode vorgesehen sein können. Vorzugsweise werden ein mittels einer EEG-Elektrode erhaltenes EEG-Signal und ein mittels einer EMG-Elektrode erhaltenes EMG-Signal der Auswerteeinheit (46) zugeführt. Diese können durch die Auswerteeinheit bei der Bestimmung eines Parameters zur transkraniellen Hirnstimulation berücksichtigt werden. Es versteht sich, dass eine EMG-Elektrode auch an anderen Elementen des TMS-Systems, wie beispielsweise in Fig. 1, angeordnet sein kann. Beispielsweise kann ein EMG Signal direkt einer Auswerteeinheit 23 in einer Stimulationseinheit 20 zugeführt sein.

Fig. 5 zeigt eine schematische Darstellung einer vorteilhaften Ausgestaltung einer Auswerteeinheit 50 gemäß einem Ausführungsbeispiel der Offenbarung. Es kann sich beispielsweise um die Auswerteeinheit 23 gemäß Fig. 1 oder die Auswerteeinheit 46 gemäß Fig. 4 handeln.

Die Auswerteeinheit 50 weist einen Eingang 51 zum Empfangen eines EEG-Signals 55 auf. Der Eingang 51 kann beispielsweise einen Verstärker sowie einen Analog-zu-Digital-Wandler (ADC) aufweisen. Das EEG-Signal 55 wird einer ersten DFT (Diskrete Fourier-Transformation)- bzw. insbesondere FFT (Fast Fourier Transform) - Einheit 52 und einer zweiten DFT bzw. FFT-Einheit 53 zugeführt. Die erste FFT-Einheit 52 ist dazu ausgebildet, eine Fourier-Analyse bzw. Fourier-Transformation des EEG-Signals durchzuführen. Dabei wird ein gleitendes Fenster des EEG-Signals betrachtet, welches eine feste Fensterbreite aufweist. In einem gewünschten Frequenzbereich, beispielsweise von 8 bis 13Hz für Alpha-Wellen oder von 13 bis 30Hz für Beta-Wellen wird eine gewünschte erste Frequenz 56 bestimmt. Dies kann beispielsweise die Peak-Frequenz in dem gewünschten Frequenzbereich sein.

Basierend auf dieser durch die erste FFT-Einheit 52 bestimmten ersten Frequenz 56 wird die Breite des Fensters für die zweite FFT-Einheit 53 derart gewählt, dass sie einem ganzzahligen Vielfachen der Wellenlänge entsprechend der ersten Frequenz 56 entspricht. Ferner wird die Breite des zweiten FFT-Fensters kleiner als die Breite des ersten FFT-Fensters gewählt. Gegenüber dem Ergebnis der Fourier-analyse mit der ersten FFT-Einheit 52 können dadurch Artefakte reduziert bzw. vermieden und eine präzisere Phasenbestimmung des EEG-Signals bei der gewünschten Frequenz erreicht werden.

Die derart bestimmte Phase 57 wird einer Triggersignal-Erzeugungseinheit 54 zugeführt, welche ein Triggersignal 58 erzeugt und an einem Ausgang bereitstellt. Der Ausgang kann wiederum mit einem externen Triggereingang 26 einer Stimuluseinheit 20, wie in Fig. 1 dargestellt, verbunden werden.

Fig. 6 zeigt ein entsprechendes Flussdiagramm zur Auswertung eines EEG-Signals 55 gemäß einem Ausführungsbeispiel der Offenbarung.

In Schritt 61 wird ein EEG-Signal empfangen.

In Schritt 62 erfolgt eine Bestimmung einer ersten Frequenz des EEG-Signals mittels einer ersten Fourieranalyse eines des EEG-Signals in einem ersten gleitenden Fenster. Bei der ersten Frequenz kann es sich beispielsweise um eine Frequenz mit maximaler Amplitude in einem gewünschten Frequenzband handeln, beispielsweise Alpha-Wellen als Indikator für einen Gehirnzustand.

In Schritt 63 wird eine Phase des EEG-Signals mittels einer zweiten Fourieranalyse des EEG-Signals in einem zweiten gleitenden Fenster bestimmt. Dabei ist das zweite Fenstern schmaler als das erste Fenster. Es wird also ein kürzerer Ausschnitt des EEG-Signals berücksichtigt, da insbesondere anzunehmen ist, dass dieser geringeren Schwankungen unterworfen ist. Die Breite des zweiten Fensters für die zweite Fourieranalyse wird dabei derart gewählt, dass sie einem ganzzahligen Vielfachen einer Wellenlänge entsprechend der in Schritt 62 bestimmten ersten Frequenz des EEG-Signals entspricht.

In Schritt 64 wird basierend auf der bestimmten Phase des EEG-Signals ein Triggersignal generiert, mit welchem ein Magnetpuls zur transkraniellen Magnetstimulation ausgelöst wird.

Figur 7 und Figur 8 zeigen eine Unteransicht und eine Seitenansicht einer vorteilhaften Ausführungsform einer Spulenanordnung zur transkraniellen Magnetstimulation mit einer integrierten Elektrodenanordnung mit wenigstens einer festen Elektrode und einer Mehrzahl an Federelektroden. Es versteht sich, dass eine derartige Elektrodenanordnung mit einer Kombination aus wenigstens einer festen Elektrode und wenigstens einer Federelektroden auch bei einer Elektrodenanordnung mit einem Halter, wie beispielhaft in Fig. 4 gezeigt, vorteilhaft eingesetzte werden kann.

In dem in Fig. 7 gezeigten Ausführungsbeispiel weist die Elektrodenanordnung 13 beispielsweise vier Federelektroden 73a, 73c, 73d, 73e auf, welche eine feste Elektrode 13b umgeben. Es können jedoch auch mehrere feste Elektroden und/oder eine andere Anzahl an Federelektroden vorgesehen sein. Vorzugsweise ist die feste Elektrode 13b bzw. sind die mehreren festen Elektroden in einem zentralen Bereich bzw. um einen zentralen Bereich der Magnetspule 12 herum angeordnet. Dadurch kann der Abstand zwischen Magnetspule 12 und dem Schädel 102 des Nutzers dort minimiert werden.

Wie in Fig. 8 gezeigt kann eine feste Elektrode 13b einen sehr geringen Abstand zwischen der im Gehäuse 11 angeordneten Magnetspule und dem Schädel des Nutzers 102 ermöglichen. Demgegenüber weisen Federelektroden 73a, 73c, 73d, 73e eine üblicherweise höhere Bauhöhe auf. Eine Federwirkung der Federelektroden kann auf verschiedene Art und Weise realisiert werden, beispielsweise mittels eine Spiralfeder 71d, einer Blattfeder 71e oder durch ein elastisches Material 71a, wie in Fig. 8 gezeigt.

Ein Vorteil der Federelektroden besteht darin, dass diese sich flexibel der Form des Schädels 102 des Nutzers 100 anpassen können, wie in der Seitenansicht in Fig. 8 für die Federelektroden 71a, 71d und 71e beispielhaft gezeigt. Dadurch kann die vorgeschlagenen Elektrodenanordnung 10 schnell und einfach an verschiedenen Stellen des Kopfes des Nutzers 100 positioniert werden. Die vorgeschlagene Kombination aus fester Elektrode 13b und Federelektroden 71a, 71c, 71d und 71e vereint somit die Vorteile einer zielgerichteten, effektiven transkraniellen Magnetstimulation verschiedener Hirnareale mit verbesserter Handhabung.

In einem Ausführungsbeispiel kann ein Federelement vorteilhaft derart angeordnet werden, dass es von einem zentralen Bereich der Spulenanordnung 10 weg weist. Somit kann eine möglichst geringere Beeinträchtigung eines starken Magnetfeldes in dem zentralen Bereich erreicht werden. Wie in Fig. 7 gezeigt weist Federelement 71e, beispielsweise eine Blattfeder, in den zentralen Bereich hinein, wohingegen Federelemente 71a und 71c vom zentralen Bereich der Spulenanordnung 10 weg weisen.

Die Elektroden weisen ferner Signalleitungen 72a bis 72e auf. Auch diese sind vorzugsweise derart angeordnet, dass ein zentraler Bereich der Spulenanordnung 10 gemieden wird. Insbesondere kann so eine durch den Magnetpuls induzierte Spannung auf den Signalleitungen vermindert werden.

Mit der vorgeschlagenen Lösung kann somit eine phasensynchrone EEG-basierte transkranielle Magnetstimulation mit hoher Präzision erfolgen, beispielsweise bei der Rehabilitation nach Schlaganfall, oder Behandlung von neuropathischen Schmerzen, Tinnitus oder Depression.

## Patentansprüche

1. Spulenanordnung (10) mit einem Gehäuse (11) und einer darin angeordneten Magnetspule (12) zum Erzeugen eines Magnetfeldes zur transkraniellen Magnetstimulation , wobei die Spulenanordnung (10) ferner eine an dem Gehäuse (11) angeordnete Elektrodenanordnung (13) zur Ableitung eines EEG-Signals aufweist, wobei die Magnetspule (12) und die Elektrodenanordnung (13) übereinander angeordnet sind, **dadurch gekennzeichnet, dass** die Elektrodenanordnung (13) wenigstens eine feste Elektrode (13b) und wenigstens eine Federelektrode (73) aufweist.

2. Spulenanordnung nach Anspruch 1, **dadurch gekennzeichnet, dass** das Gehäuse (11) ferner einen Handgriff (17) oder eine Haltevorrichtung aufweist.

3. Spulenanordnung nach einem der vorhergehenden Ansprüche, **dadurch gekennzeichnet, dass** die Elektrodenanordnung (13) wenigstens eine Trockenelektrode, oder eine Nasselektrode, insbesondere eine Elektrode aus einem superabsorbierenden Polymer, aufweist.

4. Spulenanordnung nach einem der vorhergehenden Ansprüche, **dadurch gekennzeichnet, dass** die Elektrodenanordnung (13) eine Mehrzahl an Federelektroden (73) aufweist, wobei die Federelektroden die wenigstens eine feste Elektrode umgeben.

5. Spulenanordnung nach einem der vorhergehenden Ansprüche, wobei die feste Elektrode (13b) bezüglich der Magnetspule (12) zentriert angeordnet ist.

6. Spulenanordnung nach einem der vorhergehenden Ansprüche, **dadurch gekennzeichnet, dass** die Spulenanordnung (10) ferner eine integrierte Auswerteeinheit (23) zur Auswertung des EEG-Signals aufweist; insbesondere wobei die Auswerteeinheit (23) dazu ausgebildet ist, um basierend auf dem EEG-Signal eine Anpassung eines Parameters der transkraniellen Magnetstimulation zu bewirken.

7. Spulenanordnung nach Anspruch 6, **dadurch gekennzeichnet, dass** die Auswerteeinheit (23, 50) dazu ausgebildet ist,
eine Phasenlage des EEG-Signals in einem vorgegebenen Frequenzband zu bestimmen,
wobei in einem ersten Schritt eine Bestimmung einer ersten Frequenz des EEG-Signals mittels einer ersten Spektralanalyse eines ersten gleitenden Fensters des EEG-Signals erfolgt
und in einem zweiten Schritt eine Phase des EEG-Signals mittels einer zweiten Fourieranalyse eines zweiten gleitenden Fensters des EEG-Signals erfolgt,
wobei das erste Fenster vorzugsweise breiter ist als das zweite Fenster, und wobei die Breite des zweiten Fensters für die zweite Spektralanalyse derart gewählt ist, dass sie einem ganzzahligen Vielfachen einer Wellenlänge entsprechend der ersten Frequenz des EEG-Signals entspricht
oder alternativ eine DFT Gleichung mit einem nicht-ganzzahligen, an die Wellenlänge entsprechend der ersten Frequenz des EEG-Signals angepasstem Multiplikationsparameter ermittelt wird.

8. Spulenanordnung nach einem der vorhergehenden Ansprüche, **dadurch gekennzeichnet, dass** die Spulenanordnung (10) eine Schutzschaltung zur Abschaltung von wenigstens einer Komponente zur Messung des EEG-Signals während eines Magnetpulses aufweist.

9. Spulenanordnung nach einem der vorhergehenden Ansprüche, **dadurch gekennzeichnet, dass** nach Erzeugen des Magnetfeldes ein Reset wenigstens einer Komponente zur Messung und/oder Verarbeitung des EEG-Signals und/oder zur Steuerung eines Parameters zur transkraniellen Magnetstimulation erfolgt.

10. Spulenanordnung nach einem der vorhergehenden Ansprüche, **dadurch gekennzeichnet, dass** die Spulenanordnung eine Referenzelektrode (47) aufweist.

11. Spulenanordnung nach einem der vorhergehenden Ansprüche, **dadurch gekennzeichnet, dass** die Spulenanordnung eine Elektrode zur Ableitung eines EMG-Signals aufweist.

12. Elektrodenanordnung (40) zur Ableitung eines EEG-Signals, wobei die Elektrodenanordnung (40) einen Halter (41) aufweist, der an das Gehäuse (11) einer Spulenanordnung (30) angepasst ist, wobei die Spulenanordnung (30) eine in dem Gehäuse (11) angeordneten Magnetspule (12) zum Erzeugen eines Magnetfeldes zur transkraniellen Magnetstimulation aufweist, und wobei die Elektrodenanordnung (40) mittels des Halters (41) an dem Gehäuse (11) der Spulenanordnung (10) anbringbar ist, derart dass die Magnetspule (12) und die Elektrodenanordnung (13) übereinander angeordnet sind, wobei die Elektrodenanordnung (13) wenigstens eine feste Elektrode (13b) und wenigstens eine Federelektrode (73) aufweist.

13. Elektrodenanordnung nach Anspruch 12, **dadurch gekennzeichnet, dass** die Elektrodenanordnung (40) eine integrierte Auswerteeinheit (46) zum Auswerten des EEG-Signals und Erzeugen eines Triggersignals aufweist.

14. System (1) zur transkraniellen Magnetstimulation mit:
- einer Spulenanordnung (10) nach einem der Ansprüche 1 bis 11 oder mit einer Spulenanordnung (30) und einer Elektrodenanordnung (40) nach einem der Ansprüche 12 bis 13;
- einem Stimulusgenerator (21) zum Erzeugen eines transkraniellen magnetischen Stimulus; und
- einer Steuereinheit (22) zum Ansteuern des Stimulusgenerators (21).

## Claims

1. Coil assembly (10) comprising a housing (11) and a magnetic coil (12) arranged therein for generating a magnetic field for transcranial magnetic stimulation, wherein the coil assembly (10) further comprises an electrode arrangement (13) arranged on the housing (11) for deriving an EEG signal, the magnetic coil (12) and the electrode arrangement (13) being arranged on top of each other, **characterized in that** the electrode arrangement (13) comprises at least one fixed electrode (13b) and at least one spring electrode (73).

2. Coil assembly according to claim 1, **characterized in that** the housing (11) further comprises a handle (17) or a holding assembly.

3. Coil assembly according to any of the preceding claims, **characterized in that** the electrode arrangement (13) comprises at least one of a dry electrode or a wet electrode, in particular an electrode made of a superabsorbent polymer.

4. Coil assembly according to any of the preceding claims, **characterized in that** the electrode arrangement (13) comprises a plurality of spring electrodes (73), wherein the spring electrodes surround the at least one fixed electrode.

5. Coil assembly according to any of the preceding claims, wherein the fixed electrode (13b) is arranged centered with respect to the magnetic coil (12).

6. Coil assembly according to any of the preceding claims, **characterized in that** the coil assembly (10) further comprises an integrated evaluation device (23) for evaluating the EEG signal, in particular wherein the evaluation device (23) is adapted to effect an adaptation of a parameter of the transcranial magnetic stimulation based on the EEG signal.

7. Coil assembly according to claim 6, **characterized in that** the evaluation device (23, 50) is configured to
determine a phase position of the EEG signal in a predetermined frequency band,
wherein in a first step a first frequency of the EEG signal is determined by means of a first spectral analysis of a first moving window of the EEG signal,
and in a second step a phase of the EEG signal is determined by means of a second Fourier analysis of a second moving window of the EEG signal,
wherein the first window is preferably wider than the second window,
and wherein the width of the second window for the second spectral analysis is selected such that it corresponds to an integer multiple of a wavelength corresponding to the first frequency of the EEG signal
or alternatively a DFT equation with a non-integer multiplication parameter adapted to the wavelength corresponding to the first frequency of the EEG signal is determined.

8. Coil assembly according to any of the preceding claims, **characterized in that** the coil assembly (10) comprises a protective circuit for disabling at least one component for measuring the EEG signal during a magnetic pulse.

9. Coil assembly according to any of the preceding claims, **characterized in that** after generating the magnetic field a reset of at least one component for measuring and/or processing of the EEG signal and/or for controlling a parameter for transcranial magnetic stimulation is carried out.

10. Coil assembly according to any of the preceding claims, **characterized in that** the coil assembly comprises a reference electrode (47).

11. Coil assembly according to any of the preceding claims, **characterized in that** the coil assembly comprises an electrode for deriving an EMG signal.

12. Electrode arrangement (40) for deriving an EEG signal, wherein the electrode arrangement (40) comprises a mount (41), adapted to the housing (11) of a coil assembly (30), wherein the coil assembly (30) comprises a magnetic coil (12) arranged in the housing (11) for generating a magnetic field for transcranial magnetic stimulation, and wherein the electrode arrangement (40) is adapted to be attached to the housing (11) of the coil assembly (10) by means of the mount (41), such that the magnetic coil (12) and the electrode arrangement (13) are arranged on top of each other, wherein the electrode arrangement (13) comprises at least one fixed electrode (13b) and at least one spring electrode (73).

13. Electrode arrangement according to claim 12, **characterized in that** the electrode arrangement (40) comprises an integrated evaluation device (46) for evaluating the EEG signal and for generating a trigger signal.

14. System (1) for transcranial magnetic stimulation comprising:
- a coil assembly (10) according to any of claims 1 to 11, or a coil assembly (30) and an electrode arrangement (40) according to any of claims 12 to 13;
- a stimulus generator (21) for generating a transcranial magnetic stimulus; und
- a control unit (22) for controlling the stimulus generator (21).

## Revendications

1. Agencement de bobine (10) comprenant un boîtier (11) et une bobine magnétique (12) disposée dans celui-ci pour générer un champ magnétique destiné à la stimulation magnétique transcrânienne,
dans lequel l'agencement de bobine (10) présente également un agencement d'électrodes (13) disposé sur le boîtier (11) et destiné à déduire un signal EEG, dans lequel la bobine magnétique (12) et l'agencement d'électrodes (13) sont disposés l'un au-dessus de l'autre,
**caractérisé en ce que** l'agencement d'électrodes (13) présente au moins une électrode fixe (13b) et au moins une électrode à ressort (73).

2. Agencement de bobine selon la revendication 1, **caractérisé en ce que** le boîtier (11) présente en outre une poignée (17) ou un dispositif de maintien.

3. Agencement de bobine selon l'une des revendications précédentes, **caractérisé en ce que** l'agencement d'électrodes (13) présente au moins une électrode sèche, ou une électrode humide, notamment une électrode constituée d'un polymère superabsorbant.

4. Agencement de bobine selon l'une des revendications précédentes, **caractérisé en ce que** l'agencement d'électrodes (13) présente une pluralité d'électrodes à ressort (73), dans lequel les électrodes à ressort entourent au moins une électrode fixe.

5. Agencement de bobine selon l'une des revendications précédentes, dans lequel l'électrode fixe (13b) est disposée de manière centrée par rapport à la bobine magnétique (12).

6. Agencement de bobine selon l'une des revendications précédentes, **caractérisé en ce que** l'agencement de bobine (10) présente également une unité d'évaluation (23) intégrée destinée à évaluer le signal EEG ; notamment dans lequel l'unité d'évaluation (23) est conçue pour permettre un ajustement d'un paramètre de la stimulation magnétique transcrânienne sur la base du signal EEG.

7. Agencement de bobine selon la revendication 6, **caractérisé en ce que** l'unité d'évaluation (23, 50) est conçue pour
déterminer une position de phase du signal EEG dans une bande de fréquence prédéterminée,
dans lequel, dans une première étape, une détermination d'une première fréquence du signal EEG est effectuée au moyen d'une première analyse spectrale d'une première fenêtre glissante du signal EEG
et, dans une deuxième étape, une phase du signal EEG est obtenue au moyen d'une deuxième analyse de Fourier d'une deuxième fenêtre glissante du signal EEG,
dans lequel la première fenêtre est de préférence plus large que la deuxième fenêtre,
et dans lequel la largeur de la deuxième fenêtre destinée à la deuxième analyse spectrale est sélectionnée de manière à ce qu'elle soit un multiple entier d'une longueur d'onde correspondant à la première fréquence du signal EEG
ou en variante, une équation DFT comprenant un paramètre de multiplication non entier adapté à la longueur d'onde correspondant à la première fréquence du signal EEG est déterminée.

8. Agencement de bobine selon l'une des revendications précédentes, **caractérisé en ce que** l'agencement de bobine (10) présente un circuit de protection destiné à désactiver au moins un composant destiné à mesurer le signal EEG pendant une impulsion magnétique.

9. Agencement de bobine selon l'une des revendications précédentes, **caractérisé en ce qu'**après que le champ magnétique a été généré, une réinitialisation d'au moins un composant destiné à mesurer et/ou à traiter le signal EEG et/ou à commander un paramètre de la stimulation magnétique transcrânienne est effectuée.

10. Agencement de bobine selon l'une des revendications précédentes, **caractérisé en ce que** l'agencement de bobine présente une électrode de référence (47).

11. Agencement de bobine selon l'une des revendications précédentes, **caractérisé en ce que** l'agencement de bobine présente une électrode destinée à déduire un signal EMG.

12. Agencement d'électrodes (40) destiné à déduire un signal EEG, dans lequel l'agencement d'électrodes (40) présente un support (41) qui est adapté au boîtier (11) d'un agencement de bobine (30), dans lequel l'agencement de bobine (30) présente une bobine magnétique (12) disposée dans le boîtier (11) pour produire un champ magnétique destiné à la stimulation magnétique transcrânienne, et dans lequel l'agencement d'électrodes (40) peut être monté au moyen du support (41) sur le boîtier (11) de l'agencement de bobine (10) de manière à ce que la bobine magnétique (12) et l'agencement d'électrodes (13) soient disposés l'un au-dessus de l'autre, dans lequel l'agencement d'électrodes (13) présente au moins une électrode fixe (13b) et au moins une électrode à ressort (73).

13. Agencement d'électrodes selon la revendication 12, **caractérisé en ce que** l'agencement d'électrodes (40) présente une unité d'évaluation (46) intégrée destinée à évaluer le signal EEG et à générer un signal de déclenchement.

14. Système (1) destiné à la stimulation magnétique transcrânienne, comprenant :
- un agencement de bobine (10) selon l'une des revendications 1 à 11 ou un agencement de bobine (30) et un agencement d'électrodes (40) selon l'une des revendications 12 à 13 ;
- un générateur de stimulus (21) destiné à générer un stimulus magnétique transcrânien ; et
- une unité de commande (22) destinée à commander le générateur de stimulus (21).
